# EUROPEAN PATENT SPECIFICATION

(11) **EP 4 272 769 B1**
(45) Date of publication and mention of the grant of the patent: **23.10.2024**
(21) Application number: 22171326.6
(22) Date of filing: 03.05.2022
(51) Int. Cl.: A61L 9/02, A61L 2/07, A61L 2/24, A61G 9/02

(54) **MACHINE FOR THE WASHING AND THERMODISINFECTION OF SANITARY ITEMS EQUIPPED WITH A DEVICE FOR CONDENSING THE STEAM COMING FROM THE WASH TANK**
MASCHINE ZUM WASCHEN UND THERMODESINFIZIEREN VON SANITÄRELEMENTEN MIT EINER VORRICHTUNG ZUM KONDENSIEREN DES AUS DEM WASCHTANK KOMMENDEN DAMPFES
MACHINE POUR LE LAVAGE ET LA THERMODÉSINFECTION D'ARTICLES SANITAIRES ÉQUIPÉE D'UN DISPOSITIF POUR LA CONDENSATION DE LA VAPEUR PROVENANT DU RÉSERVOIR DE LAVAGE

(43) Date of publication of application: 08.11.2023
(73) Proprietor: Bonferraro S.p.A., 37060 Bonferraro (VR) (IT)
(72) Inventor: GOBBI, Ezio, Roncoferraro MN (IT); OLIVIERI, Martino, Costermano sul Garda VR (IT)
(74) Representative: Concone, Emanuele

(56) References cited:
- EP-A1- 0 047 408
- EP-A1- 0 093 846
- DE-A1- 102010 006 448

## Description

The present invention relates to machines for washing and thermally disinfecting sanitary items such as bedpans and the like, and in particular to a machine equipped with a device for condensing the steam coming from the wash tank, particularly at the end of the thermodisinfection phase.

It is well known that a machine of this type works with high wash/rinse water temperatures that imply the production of a considerable amount of water vapour, which is also used in the final thermodisinfection phase by generating it in a special steam generator and then injecting it into the wash tank. Therefore, one of the problems in using this machine is to prevent a considerable amount of steam escaping when it is opened at the end of the operating cycle, as well as reducing the temperature in the wash tank so that the washed and disinfected items can be removed without the risk of scalding.

Of course, it would be possible to wait for the temperature in the wash tank to drop naturally so that the steam condenses on the walls of the wash tank and the articles cool down, but the waiting time for such spontaneous cooling is unacceptably long for a professional-type machine that is used frequently. To overcome these problems, the machine must also include a device that condenses the steam before the wash tank is opened, so as to prevent the steam from being discharged into the environment with adverse consequences for the operator and/or other machines in the environment, also due to the considerable variation in temperature and humidity.

EP 2033611 describes a machine of this type in which a compressor is used to inject cold air into the upper part of the wash tank in order to cool the articles and remove the steam. The air-steam mixture is conveyed downwards into a pipe branching upwards from the wash tank drain duct, this pipe comprising nozzles through which the air-steam mixture is sprayed with cold water from a storage reservoir, which is also used for rinse water. In this way, the steam condenses in the pipe and the water sprays and the water resulting from steam condensation are removed by gravity, as the pipe is connected to the drain duct, while the dehumidified air is discharged outside.

Another machine of this type is described in DE 102010006448, which differs from the machine described in EP 2033611 in that the steam is sucked from the top of the wash tank by means of an aspirator placed above it, and then condensed immediately downstream of the aspirator as well as in a condenser placed in the storage reservoir, again by spraying water from the storage reservoir. Other differences are found in the fact that the air is not discharged to the outside but recirculated to the wash tank via an overflow pipe from the storage reservoir, and that the water sprays and the water resulting from steam condensation are still removed by gravity but via a condenser drain pipe separate from that of the wash tank.

Both of these known solutions thus require the presence of an apparatus (compressor or aspirator) to remove the steam from the wash tank, as well as the consumption of a significant amount of water for steam condensation since this water is immediately discharged from the machine together with the water resulting from steam condensation EP0047408A1 discloses a machine for washing and thermodisinfecting sanitary items.

It is therefore an object of the present invention to provide a machine for washing and thermodisinfecting sanitary items which is free from these drawbacks. This object is achieved by means of a machine comprising a storage reservoir which also acts as a condenser and is connected to the wash tank in such a way as to achieve steam removal by means of natural convection, as well as a hydraulic circuit which enables the steam to be condensed with water which is recycled. Other advantageous features of the present machine are specified in the dependent claims.

The main advantage of the present machine is thus that it does not necessarily require a specific apparatus for removing steam from the wash tank, although in an alternative embodiment a fan may be provided to accelerate the removal.

A second significant advantage of this machine derives from the fact that the water used for steam condensation is not discharged but rather recycled, together with the water resulting from steam condensation, so that water consumption in the steam condensation phase is eliminated or at least substantially reduced.

Yet another advantage of the aforementioned machine is that the storage reservoir also acts as a steam condensing device and has a simple structure, which is economical both to manufacture and to install and requires no maintenance.

Further advantages and characteristics of the machine according to the present invention will be apparent to those skilled in the art from the following detailed description of two embodiments thereof with reference to the accompanying drawings, wherein:
Fig.1 is a schematic view of a first embodiment of a machine according to the invention, containing only the elements essential for understanding the invention, in the thermodisinfection phase;
Fig.2 is a schematic view of the machine of Fig.1, in the final phase of steam condensation; and
Fig.3 is a similar view to Fig.2 showing a second embodiment of the machine.

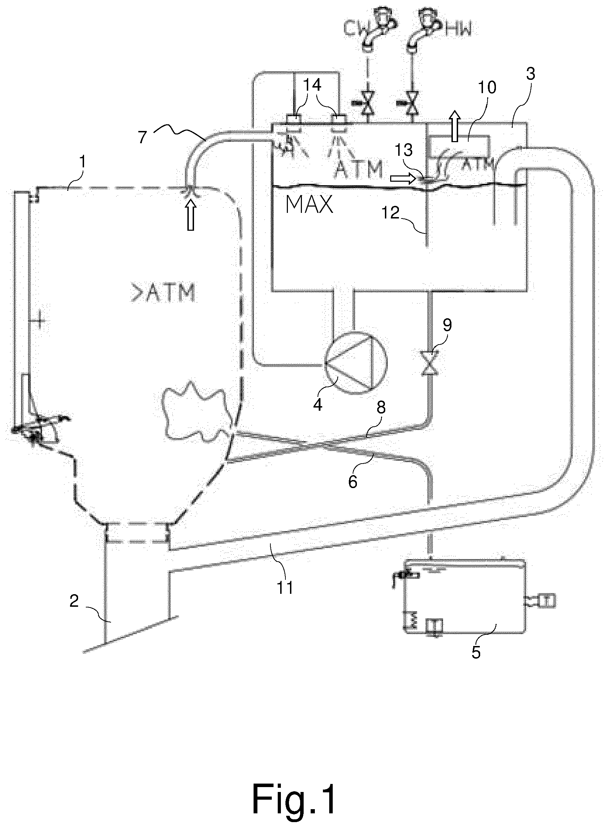

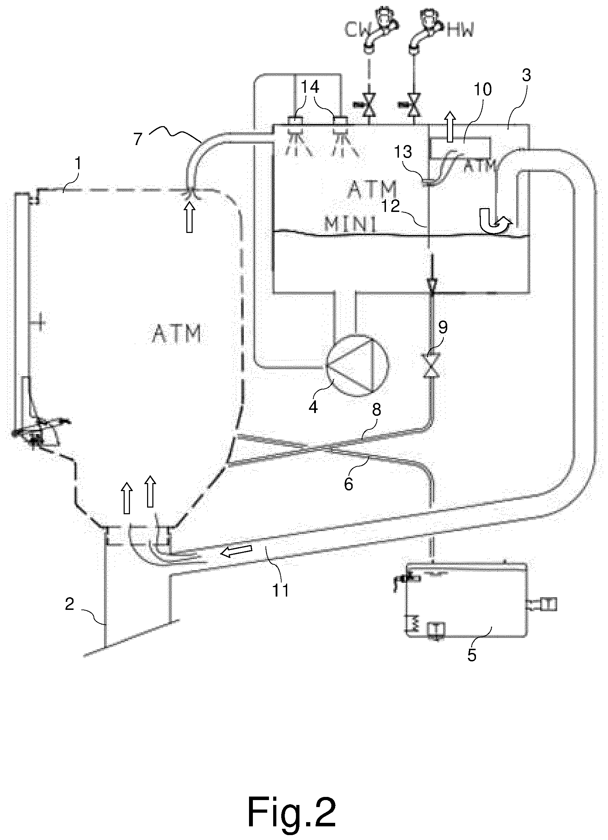

Referring to Figures 1 and 2, it can be seen that a machine according to the invention traditionally comprises:
- a wash tank 1 with a drain duct 2 extending downwards and comprising a siphon (not visible in the figures);
- a reservoir 3, located higher up than the wash tank 1, for storing the hot or cold water which is sprayed during wash and rinse by sprayers/nozzles (not visible in the figures) located in the wash tank 1 and fed by a pump 4 drawing water from the reservoir 3;
- a steam generator 5 that injects steam into the wash tank 1 through a steam pipe 6 to achieve thermodisinfection (Fig.1); and
- a controller (not shown) that manages the operation of the machine's components based on its programming and the values detected by the machine's sensors.

The innovative aspects of this machine, as mentioned above, reside in the presence of a steam condensation device that receives steam from the wash tank 1 by natural convection, and a hydraulic circuit that allows the recycling of the water used in said device. More specifically, the features that distinguish the present machine from the machines of known technique described above are:
a) reservoir 3 is connected to the wash tank 1 by means of an upper conduit 7 extending between the upper parts of said two elements, and a lower conduit 8 extending between the lower parts of said two elements and provided with a shut-off valve 9;
b) reservoir 3 is connected to the external environment through a window 10 in its upper part;
c) reservoir 3 is connected to the drain duct 2, upstream of its siphon, by means of a convection pipe 11, the upper end portion of which is shaped like an inverted siphon (i.e. in the form of an inverted U or similar) so that its end is facing downwards and located inside reservoir 3 at a lower elevation than the point where pipe 11 enters the upper part of reservoir 3;
d) reservoir 3 is divided by a partial bulkhead 12, which extends from its ceiling without reaching the bottom, into a condensation chamber into which the upper conduit 7 enters and a convection chamber into which the convection pipe 11 enters and window 10 is present;
e) bulkhead 12 is provided with an opening 13 which connects the two chambers of reservoir 3 and is located at an elevation below window 10 and above the end of the convection pipe 11, said opening 13 defining a maximum level (MAX) of the water accumulated in reservoir 3;
f) reservoir 3 is provided, in its condensation chamber, with one or more nozzles 14 fed by pump 4 and positioned to condense the steam entering through the upper conduit 7.

The wash tank 1, being connected to reservoir 3 equipped with window 10, communicates with the external environment and is therefore not to be considered a pressure vessel even when saturated with steam during the thermodisinfection phase, although a modest overpressure is in practice present. The bulkhead 12, during this thermodisinfection phase, thanks to the water present inside reservoir 3 at such a level as to realise the separation between its two chambers, prevents the steam escaping through the upper conduit 7 from passing in significant quantities to the convection chamber through opening 13 and thus from reaching the external environment through window 10. Moreover, it is also possible to condense the steam during this phase, by operating pump 4 that feeds nozzles 14 with water taken from reservoir 3 and that is not discharged but falls back into reservoir 3 itself together with the water resulting from the condensation of the steam, thus making it possible not to consume water and indeed to accumulate more water, recovering the water used in the steam generator 5.

In the final phase (Fig.2) of steam condensation after thermodisinfection, a portion of the water in the storage reservoir 3 is discharged by opening valve 9 located on the lower conduit 8, so as to lower the water level to an elevation MIN1 lower than the elevation of the end of the convection pipe 11 but corresponding to a sufficient amount of residual water to keep pump 4 running without cavitation. The closing of valve 9 can be set in various ways, for example by using a level sensor which detects when the MIN1 elevation has been reached, or by using a flowmeter which detects the amount of water which has flowed out of reservoir 3, or by calculating this amount of flowed out water on the basis of the flow rate of the lower conduit 8 and timing the closing of valve 9 accordingly.

The water drained through the lower conduit 8 is discharged to the bottom of the wash tank 1 and then reaches the drain siphon, achieving the dual effect of cooling the bottom of the wash tank 1 by lowering the temperature inside it without wetting (with non-disinfected water) the newly disinfected items, and of triggering natural convection in the wash tank 1 by opening the "water plug" that closed the convection pipe 11.

Convection motion illustrated by the arrows is now created, with the hot steam exiting the top of the wash tank 1 through the upper conduit 7 and entering the storage reservoir 3, which at this stage acts to all intents and purposes as a condenser in the condensation chamber equipped with nozzles 14. The water remaining inside reservoir 3 is recirculated by pump 4 to nozzles 14 as done previously during the thermodisinfection phase, condensing all the incoming steam, while the dehumidified air passes through opening 13 and exits through window 10. The exit of steam from the wash tank 1 generates the drawing in of external air entering through the convection pipe 11, and the condensation phase ends when the temperature inside the wash tank 1 falls below a preset value.

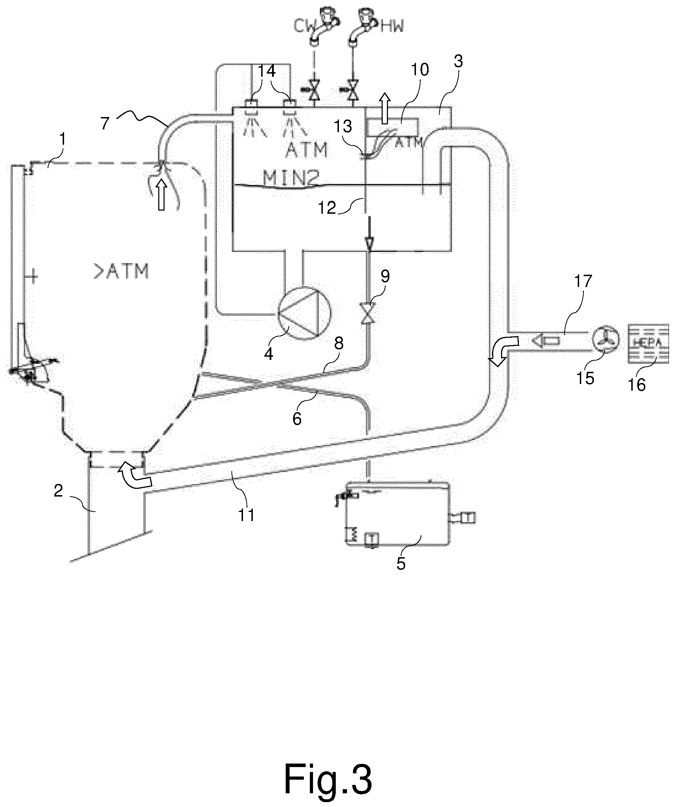

In the second embodiment illustrated in Fig.3, the steam condensing device is substantially the same but the removal of steam from the wash tank 1 is not by natural convection but by forced convection, through the introduction of external air by a fan 15, preferably preceded by a HEPA filter 16, mounted on a branch 17 of the convection pipe 11, but it could also be mounted in any other suitable position to make external air enter the wash tank 1. The entry of air creates a slight overpressure in the wash tank 1, so that the air-steam mixture exits the upper conduit 7 and the steam is condensed in reservoir 3 as explained above.

The addition of fan 15 thus does away with the first of the previously mentioned advantages, but allows the time of the steam condensation phase to be shortened since the evacuation flow rate of the wash tank 1 with forced convection is obviously greater than the flow rate with natural convection. In this respect, it should be noted that in this case opening 13 is preferably smaller in order to limit the passage through bulkhead 12, otherwise the greater flow rate entering reservoir 3 could result in a transit time in the condensation chamber insufficient to obtain a complete condensation of the steam by nozzles 14 before the air exits through window 10.

Therefore, even though opening 13 is unchanged in the figures in the two embodiments, in practice in Fig.3 it could have an area of only about 20% of that of Fig.2. Alternatively, bulkhead 12 could be provided with means for reducing the area of opening 13, so as to adapt it depending on whether fan 15 is activated or not, preferably by correlating said reduction in area to the air flow rate introduced by fan 15.

In addition, it should be noted that in this second embodiment the advantage of the recirculation of water in reservoir 3 and of the recovery of water resulting from the condensation of steam is maintained; the consumption of water is even less than in the first embodiment, since the water level is lowered to a MIN2 elevation greater than the MIN1 elevation and insufficient to open the "water plug" which closes the inlet of the convection tube 11. In this way, it is still possible to cool the bottom of the wash tank 1, and the air entering from branch 17 is conveyed to the drain duct 2, since the upper end of the convection pipe 11 remains closed.

It is clear that the embodiments of the machine according to the invention described and illustrated above are only examples susceptible to numerous variations. In particular, the shape, size and arrangement of the wash tank 1, reservoir 3, pump 4, conduits 7, 8 and pipe 11 may be varied according to constructional requirements, as long as their relative arrangement allowing the operation described above is maintained.

Finally, the method for condensing the steam present in the wash tank in the final operating phase of a machine according to the first embodiment of the present invention can be summarised in the following steps:
a) activation of pump 4 to feed nozzles 14;
b) opening of valve 9 to lower the water level in reservoir 3 to a MIN1 elevation lower than the elevation of the end of the convection tube 11 but corresponding to a sufficient residual water quantity to keep pump 4 operating without cavitation;
c) closing of valve 9 on reaching, detected or calculated, this MIN1 elevation;
d) deactivation of pump 4 when the temperature inside the wash tank 1 falls below a preset value.

Similarly, the method for condensing the steam present in the wash tank in the final phase of operation of a machine according to the second embodiment of the present invention can be summarised in the following steps:
a) activation of pump 4 to feed nozzles 14 and of fan 15;
b) opening of valve 9 to lower the water level in reservoir 3 to a MIN2 elevation above the elevation of the end of the convection tube 11;
c) closing of valve 9 on reaching, detected or calculated, this MIN2 elevation;
d) deactivation of pump 4 and fan 15 when the temperature inside the wash tank 1 falls below a preset value.

Note that in this case steps b) and c) could even be omitted if the inlet of air pushed by fan 15 were sufficient to lower the temperature in the wash tank 1 without requiring cooling with water from reservoir 3.

## Claims

1. A machine for washing and thermodisinfecting sanitary items, comprising:
- a wash tank (1) with a drain duct (2) extending downwards and comprising a siphon;
- a reservoir (3), located higher than said wash tank (1), for the storage of hot or cold water which, during the wash and rinse phases, is sprayed by sprayers and/or nozzles present in said wash tank (1) and fed by a pump (4) which draws water from said reservoir (3);
- a steam generator (5) that injects steam into the wash tank (1) through a steam pipe (6) to achieve thermodisinfection; and
- a controller that manages the operation of the machine's components based on its programming and the values detected by the machine's sensors;
wherein the reservoir (3)
a) is connected to the wash tank (1) by means of an upper conduit (7) extending between the upper parts of said two elements, and a lower conduit (8) extending between the lower parts of said two elements and provided with a shut-off valve (9);
b) is connected to the external environment through a window (10) in its upper part;
c) is connected to said drain duct (2), upstream of its siphon, by means of a convection pipe (11) the upper end of which is shaped as an inverted siphon, so that its end is facing downwards and is situated inside the reservoir (3) at a lower elevation than the point where said convection pipe (11) enters the upper part of the reservoir (3);
d) is divided by a partial bulkhead (12), extending from its ceiling without reaching the bottom, into a condensation chamber into which said upper conduit (7) enters and a convection chamber into which said convection pipe (11) enters and said window (10) is present, said partial bulkhead (12) being provided with an opening (13) which puts said two chambers of the reservoir (3) into communication with each other and is situated at an elevation below said window (10) and above the end of the convection pipe (11);
e) is fitted in its condensation chamber with one or more nozzles (14) fed by said pump (4) and positioned to condense the steam entering through the upper conduit (7).

2. Machine according to claim 1, **characterised in that** it further comprises a fan (15), preferably preceded by a HEPA filter (16), mounted in a suitable position for making external air enter the wash tank (1), said fan (15) being preferably mounted on a branch (17) of the convection pipe (11).

3. Machine according to claim 2, **characterised in that** the opening (13) has an area of about 20% of the area it would have in the absence of the fan (15).

4. Machine according to claim 2, **characterised in that** the partial bulkhead (12) is provided with means for reducing the area of the opening (13), the actuation of said means for reducing the area being preferably related to the air flow rate introduced by the fan (15).

5. A method for condensing steam present in the wash tank (1) of a machine according to claim 1, **characterised in that** it comprises the following sequential steps:
a) activation of the pump (4) to feed the nozzles (14);
b) opening of the valve (9) to lower the water level in the reservoir (3) to a (MINI) elevation lower than the elevation of the end of the convection pipe (11) but corresponding to a sufficient quantity of residual water to keep the pump (4) running without cavitation;
c) closing of the valve (9) on reaching, detected or calculated, said (MINI) elevation;
d) deactivation of the pump (4) when the temperature inside the wash tank (1) falls below a preset value.

6. A method for condensing steam present in the wash tank of a machine according to any of claims 2 to 4, **characterised in that** it comprises the following sequential steps
a) activation of the pump (4) to feed the nozzles (14) and of the fan (15);
b) opening of the valve (9) to lower the water level in the reservoir (3) to a (MIN2) elevation above the elevation of the end of the convection pipe (11);
c) closing of the valve (9) on reaching, detected or calculated, said (MIN2) elevation;
d) deactivation of the pump (4) and fan (15) when the temperature inside the wash tank (1) falls below a preset value.

## Patentansprüche

1. Maschine zum Waschen und Thermodesinfizieren von Hygieneartikeln, umfassend:
- einen Waschtank (1) mit einem Ablaufkanal (2), der sich nach unten erstreckt und einen Siphon umfasst;
- einen Behälter (3), der höher als der Waschtank (1) angebracht ist, für die Aufbewahrung von heißem oder kaltem Wasser, das, während der Wasch- und Spülphasen, durch Sprüher und/oder Düsen versprüht wird, die in dem Waschtank (1) vorhanden sind und durch eine Pumpe (4), die Wasser von dem Behälter (3) ansaugt, gespeist werden;
- einen Dampferzeuger (5), der Dampf in den Waschtank (1) über ein Dampfrohr (6) einspritzt, um eine Thermodesinfektion zu erreichen; und
- eine Steuerung, die den Betrieb der Komponenten der Maschine basierend auf seiner Programmierung und den Werten, die durch die Sensoren der Maschine erfasst werden, verwaltet;
wobei der Behälter (3)
a) mit dem Waschtank (1) mittels einer oberen Leitung (7), die sich zwischen den oberen Teilen der zwei Elemente erstreckt, und einer unteren Leitung (8) verbunden ist, die sich zwischen den unteren Teilen der zwei Elemente erstreckt und mit einem Absperrventil (9) versehen ist;
b) über ein Fenster (10) in seinem oberen Teil mit der Außenumgebung verbunden ist;
c) mit dem Ablaufkanal (2), stromaufwärts von seinem Siphon, mittels eines Konvektionsrohrs (11) verbunden ist, wobei das obere Ende davon als ein umgekehrter Siphon geformt ist, sodass sein Ende nach unten zeigt und im Inneren des Behälters (3) auf einer niedrigeren Höhe als der Punkt gelegen ist, wo das Konvektionsrohr (11) in den oberen Teil des Behälters (3) eintritt;
d) durch eine Teiltrennwand (12), die sich von seiner Decke erstreckt, ohne den Boden zu erreichen, in eine Kondensationskammer, in die die obere Leitung (7) eintritt, und eine Konvektionskammer, in die das Konvektionsrohr (11) eintritt und in der das Fenster (10) vorhanden ist, unterteilt ist, wobei die Teiltrennwand (12) mit einer Öffnung (13) versehen ist, die die zwei Kammern des Behälters (3) in Kommunikation miteinander bringt, und auf einer Höhe unterhalb des Fensters (10) und oberhalb des Endes des Konvektionsrohrs (11) gelegen ist;
e) in seiner Kondensationskammer mit einer oder mehreren Düsen (14) ausgestattet ist, die durch die Pumpe (4) gespeist werden und positioniert sind, um den Dampf, der über die obere Leitung (7) eintritt, zu kondensieren.

2. Maschine nach Anspruch 1, **dadurch gekennzeichnet, dass** sie ferner einen Ventilator (15) umfasst, dem vorzugsweise ein HEPA-Filter (16) vorangeht, der an einer geeigneten Position montiert ist, um Außenluft zu veranlassen, in den Waschtank (1) einzutreten, wobei der Ventilator (15) vorzugsweise an einem Zweig (17) des Konvektionsrohrs (11) montiert ist.

3. Maschine nach Anspruch 2, **dadurch gekennzeichnet, dass** die Öffnung (13) eine Fläche von etwa 20 % der Fläche aufweist, die sie ohne den Ventilator (15) aufweisen würde.

4. Maschine nach Anspruch 2, **dadurch gekennzeichnet, dass** die Teiltrennwand (12) mit einem Mittel zum Reduzieren der Fläche der Öffnung (13) versehen ist, wobei sich die Betätigung des Mittels zum Reduzieren der Fläche vorzugsweise auf die Luftströmungsrate, die durch den Ventilator (15) eingeführt wird, bezieht.

5. Verfahren zum Kondensieren von Dampf, der in dem Waschtank (1) einer Maschine nach Anspruch 1 vorhanden ist, **dadurch gekennzeichnet, dass** es die folgenden aufeinanderfolgenden Schritte umfasst:
a) Aktivieren der Pumpe (4), um die Düsen (14) zu speisen;
b) Öffnen des Ventils (9), um den Wasserstand in dem Behälter (3) auf eine Höhe (MIN1) zu senken, die niedriger als die Höhe des Endes des Konvektionsrohrs (11) ist, aber einer ausreichenden Restwassermenge entspricht, um die Pumpe (4) ohne Kavitation laufen zu lassen;
c) Schließen des Ventils (9) bei einem Erreichen, erkannt oder berechnet, der Höhe (MIN1);
d) Deaktivieren der Pumpe (4), wenn die Temperatur im Inneren des Waschtanks (1) unterhalb eines voreingestellten Werts fällt.

6. Verfahren zum Kondensieren von Dampf, der in dem Waschtank einer Maschine nach einem der Ansprüche 2 bis 4 vorhanden ist,
**dadurch gekennzeichnet, dass** es die folgenden aufeinanderfolgenden Schritte umfasst
a) Aktivieren der Pumpe (4), um die Düsen (14) zu speisen, und des Ventilators (15);
b) Öffnen des Ventils (9), um den Wasserstand in dem Behälter (3) auf eine Höhe (MIN2) oberhalb der Höhe des Endes des Konvektionsrohrs (11) zu senken;
c) Schließen des Ventils (9) bei dem Erreichen, erkannt oder berechnet, der Höhe (MIN2);
d) Deaktivieren der Pumpe (4) und des Ventilators (15), wenn die Temperatur im Inneren des Waschtanks (1) unterhalb eines voreingestellten Werts fällt.

## Revendications

1. Machine de lavage et de thermodésinfection d'articles sanitaires, comprenant :
- une cuve de lavage (1) dotée d'un conduit d'évacuation (2) s'étendant vers le bas et comprenant un siphon ;
- un réservoir (3), situé plus haut que ladite cuve de lavage (1), pour le stockage d'eau chaude ou froide qui, pendant les phases de lavage et de rinçage, est pulvérisée par des pulvérisateurs et/ou des buses présents dans ladite cuve de lavage (1) et alimentée par une pompe (4) qui puise l'eau à partir dudit réservoir (3) ;
- un générateur de vapeur (5) qui injecte de la vapeur dans la cuve de lavage (1) par l'intermédiaire d'un tuyau de vapeur (6) pour obtenir une thermodésinfection ; et
- un dispositif de commande qui gère le fonctionnement des composants de la machine en fonction de sa programmation et des valeurs détectées par les capteurs de la machine ;
dans laquelle le réservoir (3)
a) est relié à la cuve de lavage (1) au moyen d'une canalisation supérieure (7) s'étendant entre les parties supérieures desdits deux éléments, et d'une canalisation inférieure (8) s'étendant entre les parties inférieures desdits deux éléments et pourvu d'une soupape d'arrêt (9) ;
b) est relié à l'environnement extérieur par une fenêtre (10) située dans sa partie supérieure ;
c) est relié audit conduit d'évacuation (2), en amont de son siphon, au moyen d'un tuyau de convection (11) dont l'extrémité supérieure a la forme d'un siphon inversé, de sorte que son extrémité est orientée vers le bas et est située à l'intérieur du réservoir (3) à une élévation inférieure à celle du point où ledit tuyau de convection (11) entre dans la partie supérieure du réservoir (3) ;
d) est divisé par une cloison partielle (12), s'étendant depuis son plafond sans atteindre le fond, en une chambre de condensation dans laquelle entre ledit conduit supérieur (7) et une chambre de convection dans laquelle entre ledit tuyau de convection (11) et dans laquelle est présente ladite fenêtre (10), ladite cloison partielle (12) étant pourvue d'une ouverture (13) qui met en communication lesdites deux chambres du réservoir (3) l'une avec l'autre et qui est située à une élévation inférieure à ladite fenêtre (10) et supérieure à l'extrémité du tuyau de convection (11) ;
e) est équipé dans sa chambre de condensation d'une ou plusieurs buses (14) alimentées par ladite pompe (4) et positionnées pour condenser la vapeur entrant par la canalisation supérieure (7).

2. Machine selon la revendication 1, **caractérisée en ce qu'**elle comprend en outre un ventilateur (15), de préférence précédé d'un filtre HEPA (16), monté dans une position appropriée pour faire entrer de l'air extérieur dans la cuve de lavage (1), ledit ventilateur (15) étant de préférence monté sur une branche (17) du tuyau de convection (11).

3. Machine selon la revendication 2, **caractérisée en ce que** l'ouverture (13) a une surface d'environ 20 % de la surface qu'elle aurait en l'absence du ventilateur (15).

4. Machine selon la revendication 2, **caractérisée en ce que** la cloison partielle (12) est pourvue d'un moyen de réduction de la surface de l'ouverture (13), l'actionnement dudit moyen de réduction de la surface étant de préférence lié au débit d'air introduit par le ventilateur (15).

5. Procédé de condensation de la vapeur présente dans la cuve de lavage (1) d'une machine selon la revendication 1, **caractérisé en ce qu'**il comprend les étapes séquentielles suivantes :
a) l'activation de la pompe (4) pour alimenter les buses (14) ;
b) l'ouverture de la soupape (9) pour abaisser le niveau d'eau dans le réservoir (3) à une élévation (MIN1) inférieure à l'élévation de l'extrémité du tuyau de convection (11) mais correspondant à une quantité d'eau résiduelle suffisante pour maintenir la mise en oeuvre de la pompe (4) sans cavitation ;
c) la fermeture de la soupape (9) lorsqu'elle atteint ladite élévation (MIN1 ), détectée ou calculée ;
d) la désactivation de la pompe (4) lorsque la température à l'intérieur de la cuve de lavage (1) tombe sous une valeur prédéfinie.

6. Procédé de condensation de la vapeur présente dans la cuve de lavage d'une machine selon l'une quelconque des revendications 2 à 4,
**caractérisé en ce qu'**il comprend les étapes séquentielles suivantes
a) l'activation de la pompe (4) pour alimenter les buses (14) et du ventilateur (15) ;
b) l'ouverture de la soupape (9) pour abaisser le niveau d'eau dans le réservoir (3) à une élévation (MIN2) supérieure à l'élévation de l'extrémité du tuyau de convection (11) ;
c) la fermeture de la soupape (9) lorsqu'elle atteint ladite élévation (MIN2), détectée ou calculée ;
d) la désactivation de la pompe (4) et du ventilateur (15) lorsque la température à l'intérieur de la cuve de lavage (1) tombe sous une valeur prédéfinie.
